# EUROPEAN PATENT SPECIFICATION

(11) **EP 2 068 762 B1**
(45) Date of publication and mention of the grant of the patent: **27.06.2012**
(21) Application number: 07837742.1
(22) Date of filing: 05.09.2007
(51) Int. Cl.: A61F 2/06, A61F 2/90

(54) **INTRA-COLUMNAR CELL FEATURES TO IMPROVE DRUG DISTRIBUTION AND SCAFFOLDING OF A STENT**
SÄULEN-ZELLFUNKTIONEN ZUR VERBESSERUNG DER VERBREITUNG VON WIRKSTOFFEN UND DES GERÜSTS EINES STENTS
CARACTÉRISTIQUES DE CELLULES INTRA-COLONNES PERMETTANT D'AMÉLIORER LA DIFFUSION MÉDICAMENTEUSE ET LA STRUCTURE D'UNE ENDOPROTHÈSE

(30) Priority: 12.09.2006 US 843873 P; 24.08.2007 US 844583
(43) Date of publication of application: 17.06.2009
(73) Proprietor: Boston Scientific Limited, Barbados, West Indies (BB)
(72) Inventor: DAVIS, Michael, Rockford, Minnesota 55373 (US); BROWN, Brian, Hanover, Minnesota 55341 (US)
(74) Representative: Schildberg, Peter
(86) International application number: PCT/US2007/019363
(87) International publication number: WO 2008/033245

(56) References cited:
- US-A- 5 931 867
- US-A1- 2001 044 652
- US-A1- 2003 120 336
- US-B1- 6 440 162
- US-B1- 7 090 694

## Description

### BACKGROUND OF THE INVENTION

A stent is a medical device introduced to a body lumen and is well known in the art Typically, a stent is implanted in a blood vessel at the site of a stenosis or aneurysm endoluminally, i.e. by so-called "minimally invasive techniques" in which the stent in a radially reduced configuration, optionally restrained in a radially compressed configuration by a sheath and/or catheter, is delivered by a stent delivery system or "introducer" to the site where it is required. The introducer may enter me body from an access location outside the body, such as through the patient's skin, or by a "cut down" technique in which the entry blood vessel is exposed by minor surgical means.

Stents, grafts, stent-grafts, vena cava filters, expandable frameworks, and similar implantable medical devices, collectively referred to hereinafter as stents, are radially expandable endoprostheses which are typically intravascular implants capable of being implanted transluminal and enlarged radially after being introduced percutaneously. Stents may be implanted in a variety of body lumens or vessels such as within the vascular system, urinary tracts, bile ducts, fallopian tubes, coronary vessels, secondary vessels, etc. They may be self-expanding, expanded by an internal radial force, such as when mounted on a balloon, or a combination of self-expanding and balloon expandable (hybrid expandable). An example of a balloon expandable stent is shown in U.S. Pat No. 5843120. An example of a self-expanding stent is described in WO 96/26689.

Stents may be created by methods including cutting or etching a design from a tubular stock, from a flat sheet which is cut or etched and which is subsequently rolled or from one or more interwoven wires or braids.

US 2003/0120336 A1 discloses a stent with a dual support structure. The known stent comprises beams having an undulating pattern to define a plurality of peaks and valleys forming cells. Slots are formed through the complete thickness of each of the beams. The slots extend between first and second ends, wherein first ends are adjacent to the longitudinal connection between two adjacent beams. The slots comprise a width which remains constant throughout its length. Upon expansion of the stent the cells assume a diamond-like shape while the width of the stent remains constant.

US 7,090,694 B1 discloses a portal design for stent for treating bifurcated vessels. The bifurcating has an improved portal region between a proximal section and a distal section with the stent. The bifurcated stent can be expanded to a greater diameter than the remaining stent. In one example of the known bifurcated stent the portal region 28 is formed of nested peaks nested within adjacent peaks to form the portal region (cp. column 10, lines 10 - 19).

US 6,440,162 B1 discloses an expandable stent with arms having expandable slits in the substantially straight arms.The problem of the invention is to provide stent patterns that provide proper scaffolding support and drug delivery in the expanded state, while also allowing for crimpability and for flexibility and deliverability in the unexpanded state.

The problem is solved by a stent according to claim 1.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

The present invention is directed toward a stent as disclosed in the appended claims having a plurality of axially spaced serpentine bands, each serpentine band having an axis circumferentially oriented around the longitudinal axis of the stent. The serpentine bands have a plurality of struts spaced along the axis of the serpentine band forming alternating peaks and troughs. The serpentine bands are interconnected via a plurality of interconnecting struts to form a plurality of cells defined by axially adjacent serpentine bands and circumferentially adjacent interconnecting struts. When the stent is in its unexpanded state, each serpentine band comprises a plurality of slits within the serpentine band, each slit being non-linear and continuous from a first end to a second end. Upon expansion of the stent to its expanded state, each of the slits expands in size to form an intra-columnar cell (ICC).

According to the invention, each slit, from its first end to its second end, is formed in at least a portion of each of three consecutively connected struts.

The slits are formed in three consecutively connected struts in a serpentine band. The three connected struts include a first strut, a second strut and third strut The first, second and third struts each have a first end and a second end. The second end of the first strut is connected to the first end of the second strut and the second end of the second strut is connected to the first end of the third strut. The first end of the slit is positioned in the first strut and the second end of the slit is positioned in the third strut.

In some embodiments, the slit in the three consecutive struts crosses the axis of the serpentine band at least two times. In some embodiments, the slit crosses the axis of the serpentine band three times.

In some embodiments, the slits are formed in three consecutively connected struts in a serpentine band. The three connected struts include a first strut, a second strut and third strut Each of the first, second and third struts have a first segment and a second segment, wherein the slit separates the first segment and second segment of the each of the first, second and third struts.

In some embodiments, the first segment of the first strut is connected to the first segment of the second strut forming a trough, the first segment of the second strut is connected to the first segment of the third strut forming a peak, the second segment of the first strut is connected to the second segment of the second strut forming a peak and the second segment of the second strut is connected to the second segment of the third strut forming a trough.

Upon expansion of the stent to its expanded state, each of the slits expands in size to form an intra-columnar cell (ICC). The ICC is substantially a polygon and has at least two inner reflex angles. In some embodiments, the ICC has at least two inner acute angles. In some embodiments, the ICC has at least four inner angles less than 180°.

In some embodiments, the first and second segments of the first, second and third struts define the ICC and the first segment of the first strut is substantially parallel with the second segment of the third, strut. In some embodiments, the second segment of the first strut is substantially parallel with the first segment of the third strut. In some embodiments, the first segment of the second strut is substantially parallel with the second segment of the second strut.

In some embodiments, the second segment of the first strut and the first segment of the third strut have greater widths than the first segment of the first strut and the second segment of the third strut. In some embodiments, the first segment and second segment of the second strut may vary in their width along their lengths.

The serpentine bands may also comprise a plurality of primary hinge points, wherein, upon expansion of the stent and the forming of the ICC, the first and second segments of the first, second and third struts rotate around the primary hinge points, increasing the size of the slit to form the ICC. In some examples, a first primary hinge point is located in an end of the first segment of the second strut, wherein the first segment of the second strut and the first segment of the third strut pivot around the first primary hinge point A second primary hinge point may be located in an end of the second segment of the second strut, wherein the second segment of the second strut and the second segment of the first strut pivot around the second primary hinge point There also may be six primary hinge points.

In some embodiments, the at least a portion of the stent is configured to include one or more mechanisms for the delivery of a therapeutic agent. Often the agent will be in the form of a coating or other layer (or layers) of material placed on a surface region of the stent and is adapted to be released at the site of the stent's implantation or areas adjacent thereto. The therapeutic and/or polymeric coatings may comprise one or more non-genetic therapeutic agents, genetic materials and cells and combinations thereof.

In an example of the inventive stent is mounted on a stent delivery catheter. The present invention provides examples of methods of delivering the disclosed inventive stents to a target site in a bodily vessel.

These and other embodiments that characterize the invention are pointed out with particularity in the claims annexed hereto. However, for further understanding of the invention, its advantages and objectives obtained by its use, reference should be made to the drawings and the accompanying descriptive matter, in which there is illustrated and described an embodiments of the invention.

### BRIEF DESCRIPTION OF THE DRAWINGS

A detailed description of the invention is hereafter described with specific reference being made to the drawings.

FIG. 1 is a perspective view of an embodiment of the invention.

FIG. 2 is an expanded view of a portion 2 of the embodiment of FIG. 1.

FIG. 2A is an expanded view of a portion 2 of the embodiment of FIG. 1.

FIG. 3 is an expanded view of a portion 2 of the embodiment of FIG. 1 in its expanded state.

FIG. 4 is an expanded view of a portion of the embodiment of FIG. 1.

FIG. 5 is a partial view illustrating an embodiment of the invention.

FIG. 6 is a partial view illustrating an example which does not form a part of the invention as claimed.

FIG. 7 is a partial view illustrating an example which does not form a part of the invention as claimed.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

Depicted in the figures are various aspects of the invention. Elements depicted in one figure may be combined with, or substituted for, elements depicted in another figure as desired.

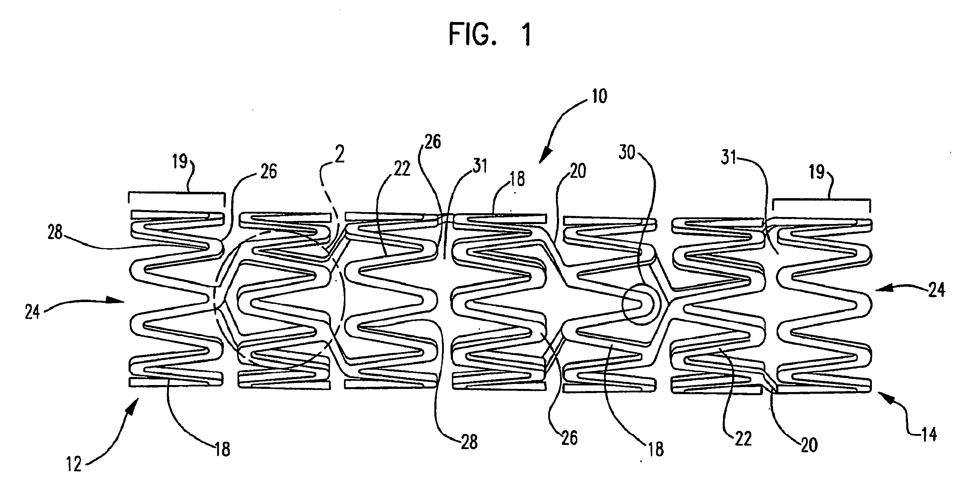

In one embodiment, as shown in FIG. 1, the invention is directed to a stent, shown generally at 10, having a proximal end 12 and a distal end 14, and comprising a plurality of axially spaced serpentine bands 18. Each serpentine band 18 is shaped in a tubular form forming a plurality of expansion columns 19. Each serpentine band 18, and therefore each expansion column 19, is connected to a longitudinally adjacent serpentine band 18 via a plurality of interconnecting struts 20. The interconnecting struts shown are of substantially the same length and arranged in a uniform patter. However, it should be understood that the interconnecting struts 20 may vary in design, number, length and pattern.

A plurality of cells 31 are defined by longitudinally adjacent serpentine bands 18 and circumferentially adjacent interconnecting struts 20. It should be understood that the shape of the cells vary and the cell pattern may be uniform or irregular.

The serpentine bands 18 comprise struts 22 circumferentially arranged around the longitudinal axis 24 of the stent 10. Adjacent struts 22 are connected to one another forming alternating peaks 26 and troughs 28. Since the junctures 30 between adjacent struts 22 could be considered to form a peak 26 and a trough 28 from a top view, the alternating peaks 26 and troughs 28 characteristic should be considered from a proximal end to a distal end or a distal end to a proximal end perspective. It should be understood that the present invention contemplates other generally serpentine configurations and not just the exact configuration shown.

The stent 10 has a contracted condition, as shown in figure 1, and an expanded condition. When the stent 10 is expanded, the diameters of the expansion columns 19 increase, the distance between circumferentially adjacent peaks 26 increases and the troughs become more obtuse.

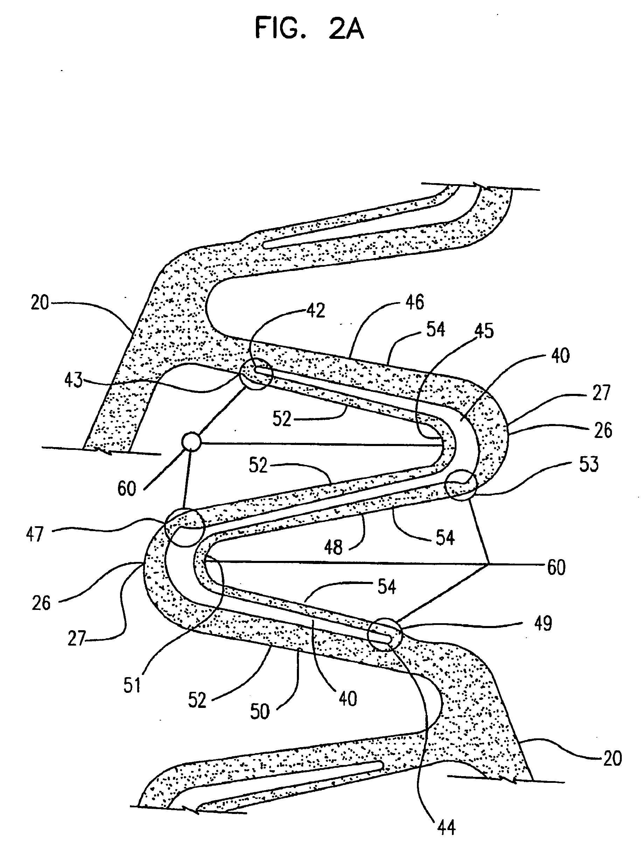

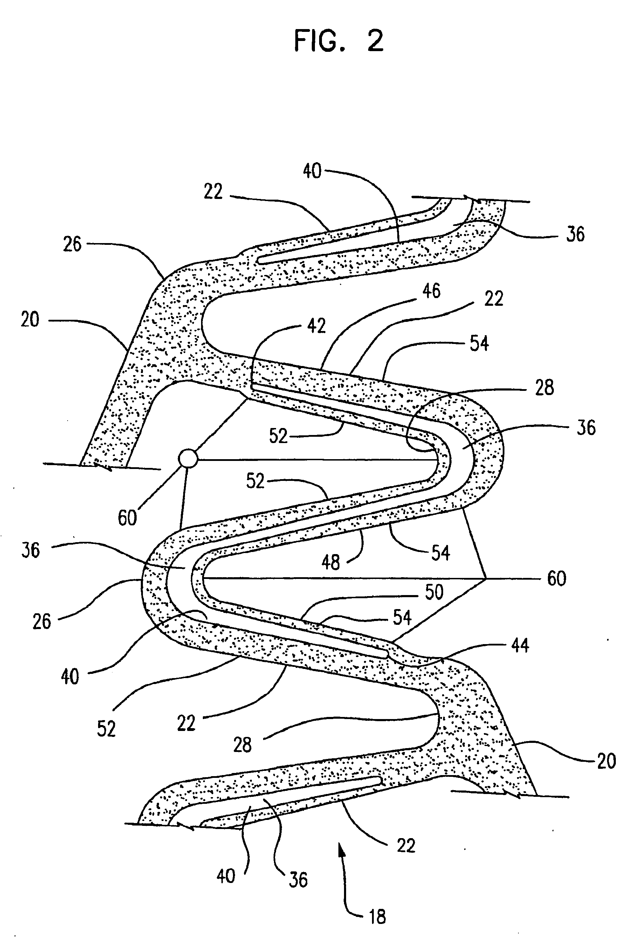

Figure 2 is an expanded view of a portion 2 of the stent of figure 1. As can be seen from this illustration of a portion of a non-expanded serpentine band 18, the serpentine bands 18 further include intra-columnar cells (ICC) 36. The ICC's 36 are intra-columnar in that they are within the serpentine band 18 as opposed to between the bands 18, as with cells 31. The ICC's 36 are visible in figure 2, however it should be understood that, when the stent 10 is in its unexpanded state, as shown in figure 2, the framework that defines and ICC 36 is contracted to such an extent that the ICC's 36 constitute slits 40 comprising very little or no open space. These slits 40 are long narrow non-linear cuts or openings fully through the serpentine bands 18 separating adjacent segments 52/54 of a strut 22. As mentioned above, the slits 40 may comprise a visual opening, but may be closed down to such an extent that the segments 52/54 of a particular strut 22 that partially define a slit 40 touch one another. They may be formed by cutting, for example laser cutting, or by any other suitable manner.

As shown in figure 2, when the stent 10 is in its contracted state, the ICC's 36 are slits 40. The slits 40 have a first end 42 continuously extending to a second end 44. In the embodiment shown in figure 2, within a single serpentine band 18, the first end 42 of the slit 40 starts in a first strut 46, continues in an adjacent second strut 48 and terminates at the second end 44 in a consecutive third strut 50. The slits 40 take on a backward or forward "S" shape as viewed when the stent 10 is horizontal, as shown in figure 1. In some embodiments, the pattern along the serpentine band is an alternating pattern of backward and forward "S" shapes.

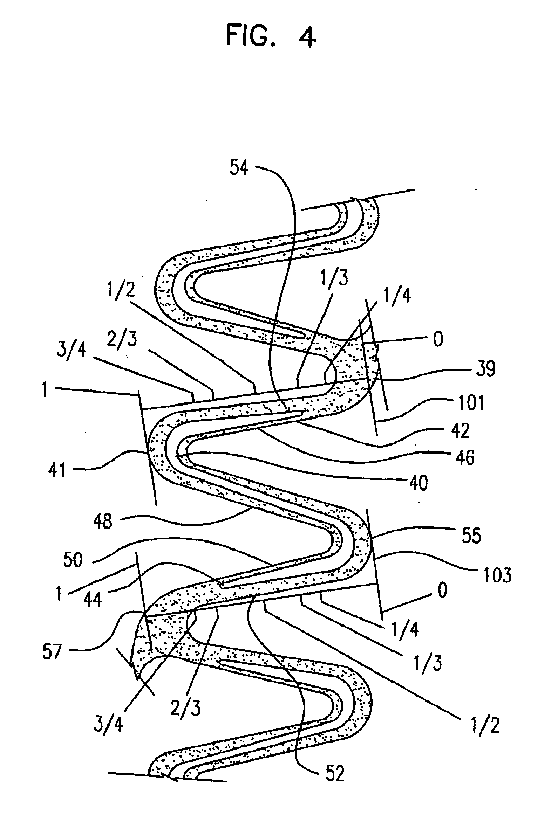

Figure 4 shows a portion of a serpentine band 18 with first strut 46, second strut 48 and third strut 50. The slit 40 shown in figure 4 is a forward "S" shape as opposed to the backward "S" shape shown in figure 2-3. The description is the same, differing only in that it is a mirror image. The slit 40 extends from its first end 42 in strut 46 to its second end 44 in strut 50. A ruler 101 is shown extending from a first end 39 of strut 46 to a second end 41. The ruler 101 is position parallel with segment 54 of the strut 46. A ruler 103 is also shown extending from a first end 55 of strut 50 to a second end 57. The ruler 103 is position parallel with segment 52 of the strut 50.

Both rulers 101, 103, are squared off at the ends 39, 41, 55, 57, of the measured struts 46, 50. The rulers 101, 103, start at 0 and go to 1, which indicates the complete length. The intermediate hashes are proportional measurements. For example, the length of strut between 0 and ½ indicates the first or beginning half of the strut and, similarly, the length of strut between ½ and 1 indicates the second or last half of the strut.

In the embodiment shown, using the rulers 101, 103, the slit 40 starts 42 in the first 1/3 of strut 46 and ends 44 in the last 1/3 of strut 50. Specifically, the start 42 point is between about % and about 1/3 of strut 46 and the end 44 is between about 2/3 and about ¾ of strut 50. In some embodiments, the slit 40 may start 42 in the first 1/4 (between and including 0 and 1/4) of strut 46 and may end 44 in the last 1/4 (between and including 3/4 and 1) of strut 50. In some embodiments, the slit 40 may start 42 in the first 1/3 (between and including 0 and 1/3) of strut 46 and may end 44 in the last 1/3 (between and including 2/3 and 1) of strut 50. In some embodiments, the slit 40 may start 42 in the first ½ (between and including 0 and ½) of strut 46 and may end 44 in the last ½ (between and including ½ and 1) of strut 50. In some embodiments, the slit 40 may start 42 in the first 2/3 (between and including 0 and 2/3) of strut 46 and may end 44 in the last 2/3 (between and including 1/3 and 1) of strut 50. In some embodiments, the slit 40 may start 42 between the first ¼ and final ¼ (between and including ¼ and 3/4) of strut 46 and may end 44 between the first ¼ and final 1/4 (between and including 1/4 and 3/4) of strut 50.

The present invention also contemplates different combinations of the starting 42 and ending 44 points mentioned above. For example, in some embodiments, the slit 40 may start in the first 1/3 (between and including 0 and 1/3) of strut 46 and may end 44 in the last ½ (between and including ½ and 1) of strut 50, etc. The invention also contemplates starting points 42 and ending points 44 between and including the specific individual hash marks shown on the rulers 101, 103. For example, between and including ½ and 1/3; between and including ½ and 2/3; between and including 1/3 and 2/3, etc. The size and opening properties of the ICC 36 can be controlled by adjusting the locations of the starting point 42 and ending point 44 of the slit 40.

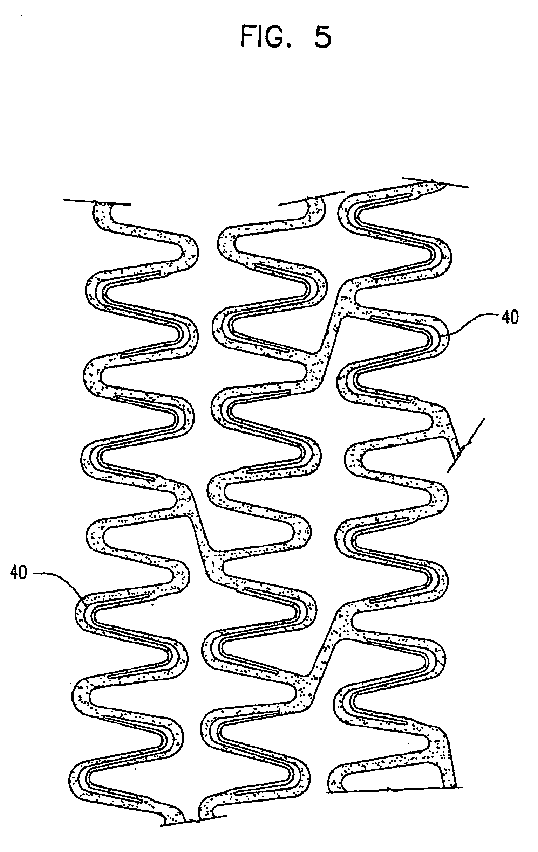

The slit 40, shown in figure 2, splits the first 46, second 48 and third 50 struts into respective first 52 and second 54 segments. When the stent 10 is in its non-expanded form, as shown in figure 2, each first segment 52 is immediately adjacent to its corresponding second segment 54. Each serpentine band 18 may contain a plurality of slits 40. In some embodiments, the slits 40 may be uniformly arranged and, in some embodiments, the slits 40 may be arranged in a non-uniform manner. A non-limiting example of a pattern of slits 40 of an embodiment of the invention is shown in figure 5. It should be understood that the pattern may vary.

Figure 2A is identical to figure 2 except that it is slightly enlarged for further illustration. In this figure 2A, primary hinge points 60 are shown in the serpentine band 18 along the slit 40. In the embodiment shown, there are six primary hinge points (PHP). As can be seen in figure 2A, a first PHP 43 is located at the end of segment 52 of strut 46 at the first end 42 of the slit 40. A second PHP 45 is located in the trough 28 between segment 52 of strut 46 and segment 52 of strut 48. A third PHP 47 is located in the end of segment 52 of strut 48 adjacent to the peak 26 between segment 52 of strut 48 and segment 52 of strut 50. PHP 47 is offset from the apex 27 of the peak 26 on the side of segment 52 of strut 48. A fourth PHP 49 is located at the end of segment 54 of strut 50 at the second end 44 of the slit 40. A fifth PHP 51 is located in the trough 28 between segment 54 of strut 48 and segment 54 of strut 50. And a sixth PHP 53 is located in the end of segment 54 of strut 48 adjacent to the peak 26 between segment 54 of strut 48 and segment 54 of strut 46. PHP 53 is offset from the apex 27 of the peak 26 on the side of segment 54 of strut 48. Upon expansion of the stent 10, the segments (52, 54) of the struts (46, 48, 50) associated with the slit 40/ICC 36, rotate around the primary hinge points 60.

The PHP may be thinned spots where plastic deformation may occur upon expansion. Positioning and design of the hinge points may also be designed using Finite Element Analysis to create stress risers that are not readily noticeable to dictate the PHP. The hinge points are located at or near the vertices of the polygon ICC structure. As the ICC structure is enlarged, the vertices of the ICC structure hinge and deform to allow the polygon shape to enlarge.

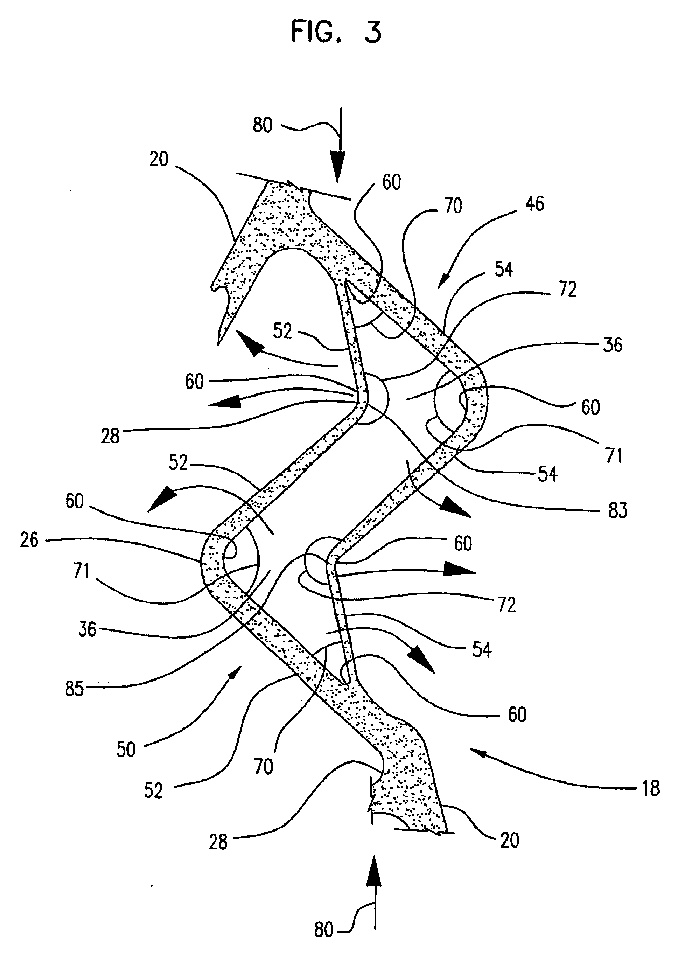

Upon radial expansion of the stent 10, as seen in figure 3, the ICCs 36 increase in area from a slit 40 to a full ICC 36. The arrows in figure 3 show the direction of movement of the segments 52, 54, of the struts 46, 48, 50, that define the ICC 36. The lengthening of the serpentine bands 18 in a circumferential direction causes the rotation of the segments 52, 54, of the struts 46, 48, 50, around the primary hinge points 60.

Upon expansion, the resulting ICC 36 shape is polygonal. The specific inner angle sizes and arrangements may vary upon deployment It will open up as needed per the final deployed diameter. A stent deployed to 5 mm may have greater angles than the same stent deployed to 4 mm.

In some embodiments, the polygonal ICC 36 has six sides and has two inner reflex angles 72, two inner acute angles 70 and two inner angles 71 that may be acute, obtuse or right. Inner angles 71 may be about 90 degrees (plus or minus 15 degrees). Although some of the inner "corners" of the ICC polygon are rounded, such as with the inner portions of the peaks 26 and/or valleys 28, they are to be considered to be inner corners of a polygon.

As can be seen in figure 3, upon expansion of the stent 10, the segments 52, 54, of the struts 46, 48, 50, move in a direction of aligning 80 with the serpentine band 18 relative to their positions when the stent 10 is in an unexpanded state, such as in figure 2. When the stent is expanded and the ICC's 36 are expanded, as shown in figure 3, the first segment 52 of strut 46 and the second segment 54 of strut 50 are largely aligned with the serpentine band 18. In this, it is meant that they are at an angle 81 of less than 45° with the axis 80 of the serpentine band. Also, when the ICC's 36 are fully expanded, the juncture 83 between segment 52 of strut 46 and segment 52 of strut 48 and the juncture 85 between segment 54 of strut 48 and segment 54 of strut 50 are substantially aligned with the axis 80 of the serpentine band 18.

Also, as can be seen in figure 3, segment 52 of strut 46 is substantially parallel with and distal to (relative to the length of the stent) segment 54 of strut 50. Segment 54 of strut 46 is substantially parallel with segment 52 of strut 50 and segments 52 and 54 of strut 48 are substantially parallel to one another. In some embodiments, these relative positions are substantially maintained from the ICC's 36 unexpanded state, as shown in figure 2, to their expanded state, as shown in figure 3.

In the embodiment shown in the figures, with regard to the linear portions of the segments, segment 52 of strut 46 and segment 54 of strut 50 are substantially the same length and are both shorter than the remaining segments of struts 46, 48 and 50. Segments 52 and 54 of strut 48 may be substantially the same length and segment 54 of strut 46 and segment 52 of strut 50 may be substantially the same length.

In some embodiments of the inventive stent, as shown in figure 3, segment 52 of strut 46 and segment 54 of strut 50 have widths that are less than that of their corresponding paired segments. The widths of both segments 52 and 54 of struts 46 and 50 may also be substantially uniform along their linear portions. The widths of segments 52 and 54 of strut 48 may have widths that inversely vary along their linear portion lengths. Segment 52 of strut 48 increases in width from its connection with segment 52 of strut 46 to its connection to segment 52 of strut 50 and segment 54 inversely decreases in width from its connection to segment 54 of strut 46 to its connection to segment 54 of strut 50. The invention also contemplates theses various thickness designs without noticeable hinge 60 thinning. The parameters may be designed to optimize the opening characteristics of the ICC structure and dependant upon the specifics of the particular stent design and ICC structure which is incorporated into the stent.

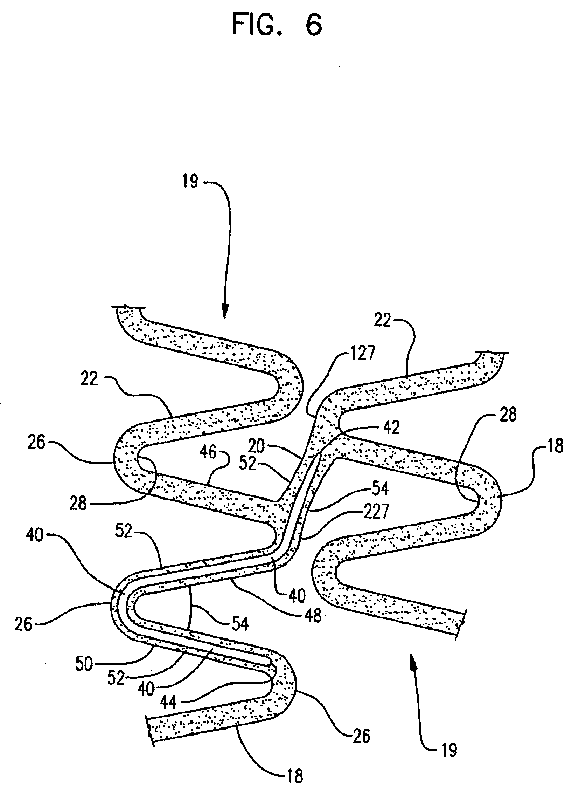

In an example which does not form a part of the invention as claimed, as shown in figure 6, instead of the first end 42 of a slit 40 being located in the first strut 46, it 42 is located in a interconnecting strut 20. The interconnecting strut 20 is measured from the apex 127 of the peak 26 of the adjacent band 18 to which the interconnecting strut 20 is connected to the apex 227 of the peak 26 that is formed from the first strut 46 and the second strut 48. The length of the interconnecting strut 20 is defined by the distance between apex 127 and apex 227. In some embodiments, the first end 42 is located in the first half of the interconnecting strut 20 starting at apex 127. In some examples, the first end 42 is located in the first third of the interconnecting strut 20 starting at apex 127. The ending 44 positioning of the slit 40 may be as described above in reference to figure 4.

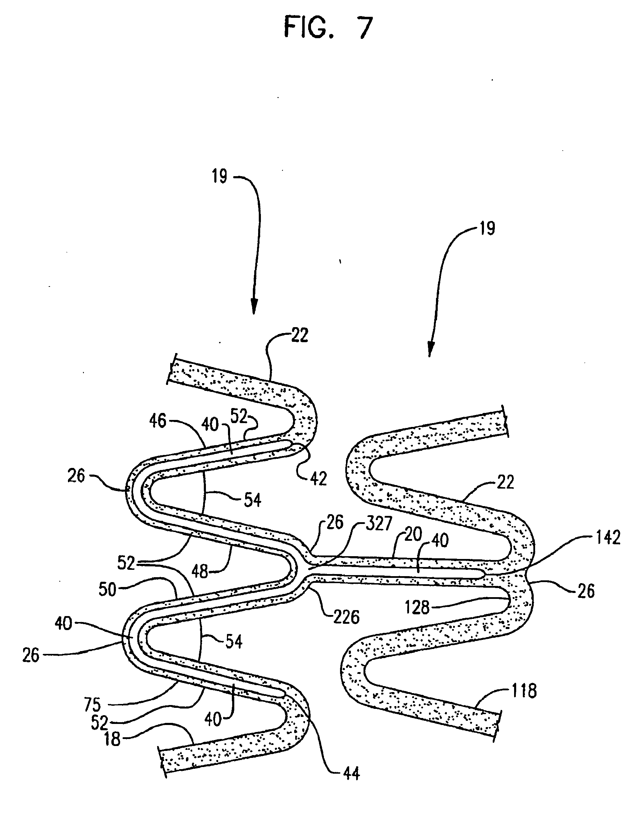

In an example which does not form a part of the invention as claimed, as shown in figure 7, the slit 40, as shown and measured in figure 4, may extend into an interconnecting strut 20 to a third end 142. In this particular embodiment, the interconnecting strut 20 is a peak 226 to trough or valley 128 interconnecting strut, however it could be a peak to peak strut. Although the slit 40 may terminate 44 in the third strut 50, as shown in figure 4, in some examples, as shown in figure 7, it 40 may extend into a fourth strut 75. The location of the second end 44 in this case is positioned in the fourth strut 75 at the same place that it would be in the third strut 50, as described above in reference to figure 4.

In the example which does not form a part of the invention shown in figure 7, the interconnecting strut 20 is measured from the valley 128 of the adjacent band 118 to which the interconnecting strut 20 is connected to the virtual apex 327 of the peak 26 that is formed from the second strut 48 and the third strut 50. It should be understood that in some examples that the interconnecting strut 20 may extend from the peak 26 that is formed from the first 46 and second 48 struts and that in some examples that the interconnecting strut 20 may extend from the peak 26 that is formed from the third 50 and fourth 75 struts. The length of the interconnecting strut 20 is defined by the distance between the valley 128 of the adjacent band 118 to the virtual apex 327. In some examples, the third end 142 is located in the first half of the interconnecting strut 20 starting at the valley 128. In some examples, the third end 142 is located in the first third of the interconnecting strut 20 starting at valley 128.

The present invention also contemplates stents having any, some or all of the slit 40 designs within the scope of the appended claims described herein.

In the above discussed embodiments, the inventive stents are of substantially uniform diameter. It is also possible to modify the stent patterns discussed above to prepare stents of non-constant diameter. For example, stent which taper in the expanded state may be made by decreasing the amplitude of the serpentine bands from one end of the stent to the other, or just along a desired portion of the stent. A tapered portion may be provided anywhere along the stent. For example, half of the stent, starting at one end of the stent, may be provided with a taper. Another way to achieve a tapered expanded stent is to change the stiffness of the serpentine bands and/or the connectors such that the stiffness of the serpentine bands and/or connectors varies along the length of the stent. The stiffness of the serpentine bands and/or connectors can be changed by altering length, width or thickness, adding additional stiffening material, using a chemical or mechanical means to alter the physical properties of the stent material, or applying one or a series of elastic elements about the stent

The inventive stent patterns disclosed herein may also be used in conjunction with other known stent designs to provide stents whose properties vary over the length or portions thereof The inventive slit patterns may also be used in non-serpentine stent designs, such as helix design, tri-bonate design, etc example which does not form a part of the invention.

The disclosure provides examples of methods of manufacturing a stent according to the designs disclosed herein (not part of the invention as claimed). The disclosure further provides examples of methods of delivering and expanding a stent as described herein (not part of the invention as claimed).

The inventive stents may be made from any suitable biocompatible materials including one or more polymers, one or more metals or combinations of polymer(s) and metal(s). Examples of suitable materials include biodegradable materials that are also biocompatible. By biodegradable is meant that a material will undergo breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, co-polymers of these materials as well as composites and combinations thereof and combinations of other biodegradable polymers. Other polymers that may be used include polyester and polycarbonate copolymers. Examples of suitable metals include, but are not limited to, stainless steel, titanium, tantalum, platinum, tungsten, gold and alloys of any of the above-mentioned metals. Examples of suitable alloys include platinum-iridium alloys, cobalt-chromium alloys including Elgiloy and Phynox, MP35N alloy and nickel-titanium alloys, for example, Nitinol.

The inventive stents may be made of shape memory materials such as superelastic Nitinol or spring steel, or may be made of materials that are plastically deformable. In the case of shape memory materials, the stent may be provided with a memorized shape and then deformed to a reduced diameter shape. The stent may restore itself to its memorized shape upon being heated to a transition temperature and having any restraints removed therefrom.

The present invention may be incorporated into both of the two basic types of catheters used in combination with a guide wire, commonly referred to as over-the-wire (OTW) catheters and rapid-exchange (RX) catheters. The construction and use of both over-the-wire and rapid-exchange catheters are well known in the art.

In some examples, the stent, the delivery system or other portion of the assembly may include one or more areas, bands, coatings, members, etc. that is (are) detectable by imaging modalities such as X-Ray, MRI, ultrasound, etc. In some examples at least a portion of the stent and/or adjacent assembly is at least partially radiopaque.

In some embodiments, at least a portion of the stent is configured to include one or more mechanisms for the delivery of a therapeutic agent Often the agent will be in the form of a coating or other layer (or layers) of material placed on a surface region of the stent, which is adapted to be released at the site of the.stent's implantation or areas adjacent thereto.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate.

The inventive stents may further comprise a polymer coating in addition to or in place of the therapeutic coating. Suitable polymer coating materials include polycarboxylic acids, cellulosic polymers, including cellulose acetate and cellulose nitrate, gelatin, polyvinylpyrrolidone, cross-linked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactone, polyhydroxybutyrate valerate and blends and copolymers thereof, coatings from polymer dispersions such as polyurethane dispersions, for example, BAYHDROL.RTM., fibrin, collagen and derivatives thereof, polysaccharides such as celluloses, starches, dextrans, alginates and derivatives, hyaluronic acid, squalene emulsions. Polyacrylic acid, available as HYDROPLUS.RTM. (Boston Scientific Corporation, Natick, Mass.), and described in U.S. Pat No. 5,091,205 is particularly desirable. In a particular desirable example of the invention, the polymer is a copolymer of polylactic acid and polycaprolactone.

In use, the stents disclosed herein are typically delivered via catheter to a desired bodily location. The choice of catheter will depend on the type of stent that is used and on the location to which the stent is delivered.

Any suitable method may be used to manufacture the inventive stents. For example, in addition to the methods listed above, the inventive stents may also be manufactured by preparing individual portions of the stent and connecting them to one another via welding, the use of adhesives or any other suitable joining technique. This list of manufacturing techniques is not meant to be exhaustive. Other manufacturing techniques may also be used to manufacture the inventive stents.

## Claims

1. A stent (10) having a proximal end (12), a distal end (14), a longitudinal axis extending through the proximal and distal ends (12, 14) the stent having an unexpanded state and an expanded state, the stent (10) further comprising: a plurality of axially spaced serpentine bands, each serpentine band (18) having an axis circumferentially oriented around the longitudinal axis of the stent (10) and a proximal side and a distal side, each band (18) comprising a plurality of struts, wherein adjacent struts are connected to each other forming a plurality of peaks (26) and troughs (28); a plurality of interconnecting struts (20) axially connecting the serpentine bands (18); and a plurality of cells (31) defined by axially adjacent serpentine bands (18) and circumferentially adjacent interconnecting struts (20), wherein, when the stent (10) is in its unexpanded state, each serpentine band (18) comprises a plurality of slits (40), upon expansion of the stent to its expanded state, each of the slits expands in size to form an intra-columnar cell (ICC), the proximal side and the distal side of the serpentine band (18) being bisected by the axis of the serpentine band, wherein the plurality of struts (22) are spaced along the axis of the serpentine band and wherein the peaks (26) have apex points (27) and the troughs (28) have center points, said peaks and troughs facing distally and proximally in an alternating manner, **characterized in that** each slit (40) is non-linear and continuous from a first end (42) to a second end (44) and being formed in at least a portion of each of three consecutively connected struts (46, 48, 50), the three consecutively connected struts comprise a first strut (46), a second strut (48) and third strut (50), the first, second and third struts each having a first end and a second end, wherein the second end of the first strut is connected to the first end of the second strut and the second end of the second strut is connected to the first end of the third strut and **in that** the first end (42) of the slit (40) is positioned in the first strut (46) and the second end of the slit (40) is positioned in the third strut

2. The stent of claim 1, wherein the slit (40) crosses the axis of the serpentine band at least three times.

3. The stent of claim 1, each of the first, second and third struts having a first segment (52) and a second segment (54), wherein the slit (40) separates the first segment and second segment of the each of the first, second and third struts.

4. The stent of claim 3, wherein the first segment (52) of the first strut (46) is connected to the first segment (52) of the second strut (48) forming a trough (28), the first segment of the second strut (48) is connected to the first segment of the third strut (50) forming a peak (26), the second segment of the first strut (46) is connected to the second segment of the second strut (48) forming a peak (26) and the second segment of the second strut (48) is connected to the second segment of the third strut (50) forming a trough (28).

5. The stent of claim 1 or 4, wherein the ICC (36) is substantially a polygon and has at least two inner reflex angles.

6. The stent of claim 5, wherein the ICC (36) has at least two inner acute angles.

7. The stent of claim 5, wherein the second segment of the first strut is substantially parallel with the first segment of the third strut and wherein the first segment of the second strut is substantially parallel with the second segment of the second strut.

8. The stent of claim 4, wherein the second segment of the first strut and the first segment of the third strut have greater widths than the first segment of the first strut and the second segment of the third strut.

9. The stent of claim 5, the serpentine bands (18) further comprising a plurality of primary hinge points (60), wherein, upon expansion of the stent (10) and the forming of the ICC (36), the first and second segments of the firsts second and third struts (46, 48, 50) rotate around the primary hinge points (60), increasing the size of the slit (40) to form the ICC.

10. The stent of claim 1, wherein the stent further comprises a therapeutic agent.

## Patentansprüche

1. Stent (10) mit einem proximalen Ende (12), einem distalen Ende (14), einer Längsachse, die sich durch das proximale und distale Ende (12, 14) erstreckt, wobei der Stent einen nicht expandierten und einen expandierten Zustand aufweist, wobei der Stent (10) darüber hinaus umfasst: eine Mehrzahl axial beabstandeter Serpentinenbänder, wobei jedes Serpentinenband (18) eine Achse aufweist, die um den Umfang um die Längssachse des Stents (10) ausgerichtet ist, und eine proximale Seite und eine distale Seite, wobei jedes Band (18) eine Mehrzahl Streben umfasst, wobei benachbarte Streben miteinander verbunden sind und eine Mehrzahl Spitzen (26) und Täler (28) bilden; eine Mehrzahl verbindender Streben (20), die die Serpentinenbänder (18) axial verbinden, und eine Mehrzahl Zellen (31), die durch axial benachbarte Serpentinenbänder (18) bestimmt sind, und um den Umfang benachbart verbindende Streben (20), wobei, wenn sich der Stent (10) in seinem nicht expandierten Zustand befindet, jedes Serpentinenband (18) eine Mehrzahl Schlitze (40) umfasst; bei Expansion des Stents in seinen expandierten Zustand expandiert die Größe jedes Schlitzes, um eine Säulenzelle (intra-columnar cell - ICC) zu bilden, wobei die proximale Seite und die distale Seite des Serpentinenbands (18) durch die Achse des Serpentinenbands in zwei Teile geteilt werden, wobei die Mehrzahl Streben (22) entlang der Achse des Serpentinenbands beabstandet sind und wobei die Spitzen (26) Scheitelpunkte (27) aufweisen und die Täler (28) Mittelpunkte aufweisen, wobei sich die Spitzen und Täler abwechselnd distal und proximal zugewandt sind, **dadurch gekennzeichnet, dass** jeder Schlitz (40) nicht-linear und durchgängig von einem ersten Ende (42) zu einem zweiten Ende (44) ist und in mindestens einem Abschnitt jeder der drei aufeinanderfolgend verbundenen Streben (46, 48, 50) gebildet ist; die drei aufeinanderfolgend verbundenen Streben umfassen eine erste Streben (46), eine zweite Streben (48) und eine dritte Streben (50), wobei die ersten, zweiten und dritten Streben je ein erstes Ende und ein zweites Ende aufweisen, wobei das zweite Ende der ersten Streben mit dem ersten Ende der zweiten Streben und das zweite Ende der zweiten Streben mit dem ersten Ende der dritten Streben verbunden ist und dass das erste Ende (42) des Schlitzes (40) in der ersten Streben (46) angeordnet ist und das zweite Ende des Schlitzes (40) in der dritten Strebe angeordnet ist.

2. Stent nach Anspruch 1, wobei der Schlitz (40) die Achse des Serpentinenbands mindestens dreimal kreuzt.

3. Stent nach Anspruch 1, wobei jede der ersten, zweiten und dritten Streben ein erstes Segment (52) und ein zweites Segment (54) aufweist, wobei der Schlitz (40) das erste Segment und das zweite Segment jeder der ersten, zweiten und dritten Streben trennt.

4. Stent nach Anspruch 3, wobei das erste Segment (52) der ersten Strebe (46) mit dem ersten Segment (52) der zweiten Strebe (48) verbunden ist und ein Tal (28) bildet, das erste Segmente der zweiten Strebe (48) mit dem ersten Segment der dritten Strebe (50) verbunden ist und eine Spitze (26) bildet, das zweite Segment der ersten Strebe (46) mit dem zweiten Segment der zweiten Strebe (48) verbunden ist und eine Spitze (26) bildet und das zweite Segment der zweiten Strebe (48) mit dem zweiten Segment der dritten Strebe (50) verbunden ist und ein Tal (28) bildet.

5. Stent nach Anspruch 1 oder Anspruch 4, wobei die ICC (36) im Wesentlichen ein Vieleck ist und mindestens zwei innere überstumpfe Winkel aufweist.

6. Stent nach Anspruch 5, wobei die ICC (36) mindestens zwei innere spitze Winkel aufweist.

7. Stent nach Anspruch 5, wobei das zweite Segment der ersten Strebe im Wesentlichen parallel zum ersten Segment der dritten Strebe ist und wobei das erste Segment der zweiten Strebe im Wesentlichen parallel zum zweiten Segment der zweiten Strebe ist.

8. Stent nach Anspruch 4, wobei das zweite Segment der ersten Strebe und das erste Segment der dritten Strebe größere Breiten aufweisen als das erste Segment der ersten Strebe und das zweite Segment der dritten Strebe.

9. Stent nach Anspruch 5, wobei die Serpentinenbänder (18) weiterhin eine Mehrzahl primärer Gelenkpunkte (60) umfassen, wobei sich die ersten und zweiten Segmente der ersten und dritten Streben (46, 48, 50) bei Ausdehnung des Stents (10) und der Bildung der ICC (36) um die primären Gelenkpunkte (60) drehen, wodurch die Größe der Schlitze (40) zum Bilden der ICC vergrößert wird.

10. Stent nach Anspruch 1, wobei der Stent weiterhin ein Therapeutikum umfasst.

## Revendications

1. Stent (10) ayant une extrémité proximale (12), une extrémité distale (14), un axe longitudinal s'étendant à travers les extrémités proximale et distale (12, 14), le stent ayant un état non dilaté et un état dilaté, le stent (10) comprenant également : une pluralité de bandes tortueuses espacées axialement, chaque bande tortueuse (18) ayant un axe orienté dans le sens circonférentiel autour de l'axe longitudinal du stent (10) et un côté proximal et un côté distal, chaque bande (18) comprenant une pluralité de montants, dans lequel des montants adjacents sont raccordés l'un à l'autre en formant une pluralité de pics (26) et de vallées (28) ; une pluralité de montants d'interconnexion (20) connectant axialement les bandes tortueuses (18) ; et une pluralité de cellules (31) définies par des bandes tortueuses (18) axialement adjacentes et des montants d'interconnexion (20) adjacents dans le sens circonférentiel, dans lequel, quand le stent (10) est dans son état non dilaté, chaque bande tortueuse (18) comprend une pluralité de fentes (40), lors de la dilation du stent vers son état dilaté, chacune des fentes se dilate en taille pour former une cellule intra-colonne (ICC), le côté proximal et le côté distal de la bande tortueuse (18) étant coupés par l'axe de la bande tortueuse, dans lequel la pluralité de montants (22) sont espacés le long de l'axe de la bande tortueuse et dans lequel les pics (26) ont des points de sommet (27), et les vallées (28) ont des points centraux, lesdits pics et vallées se faisant face de façon distale et proximale d'une façon alternée, **caractérisé en ce que** chaque fente (40) est non linéaire et continue à partir d'une première extrémité (42) vers une deuxième extrémité (44) et étant formée dans au moins une portion de chacun des trois montants (46, 48, 50) connectés consécutivement, les trois montants connectés consécutivement comprennent un premier montant (46), un deuxième montant (48) et un troisième montant (50), les premier, deuxième et troisième montants ayant chacun une première extrémité et une deuxième extrémité, dans lequel la deuxième extrémité du premier montant est connectée à la première extrémité du deuxième montant et la deuxième extrémité du deuxième montant est connectée à la première extrémité du troisième montant, et **en ce que** la première extrémité (42) de la fente (40) est positionnée dans le premier montant (46) et la deuxième extrémité de la fente (40) est positionnée dans le troisième montant.

2. Stent selon la revendication 1, dans lequel la fente (40) croise l'axe de la bande tortueuse au moins trois fois.

3. Stent selon la revendication 1, chacun des premier, deuxième et troisième montants ayant un premier segment (52) et un deuxième segment (54), dans lequel la fente (40) sépare le premier segment et le deuxième segment de chacun des premier, deuxième et troisième montants.

4. Stent selon la revendication 3, dans lequel le premier segment (52) du premier montant (46) est connecté au premier segment (52) du deuxième montant (48) formant une vallée (28), le premier segment du deuxième montant (48) est connecté au premier segment du troisième montant (50) formant un pic (26), le deuxième segment du premier montant (46) est connecté au deuxième segment du deuxième montant (48) formant un pic (26), et le deuxième segment du deuxième montant (48) est connecté au deuxième segment du troisième montant (50) formant une vallée (28).

5. Stent selon la revendication 1 ou 4, dans lequel l'ICC (36) est essentiellement un polygone et a au moins deux angles rentrants intérieurs.

6. Stent selon la revendication 5, dans lequel l'ICC (36) a au moins deux angles aigus intérieurs.

7. Stent selon la revendication 5, dans lequel le deuxième segment du premier montant est essentiellement parallèle au premier segment du troisième montant, et dans lequel le premier segment du deuxième montant est essentiellement parallèle au deuxième segment du deuxième montant.

8. Stent selon la revendication 4, dans lequel le deuxième segment du premier montant et le premier segment du troisième montant ont des largeurs plus grandes que le premier segment du premier montant et le deuxième segment du troisième montant.

9. Stent selon la revendication 5, les bandes sinueuses (18) comprenant également une pluralité de points d'articulation primaires (60), dans lequel, lors de la dilation du stent (10) et de la formation de l'ICC (36), les premier et deuxième segments des premier, deuxième et troisième montants (46, 48, 50) tournent autour des points d'articulation primaires (60), augmentant la taille de la fente (40) pour former l'ICC.

10. Stent selon la revendication 1, dans lequel le stent comprend également un agent thérapeutique.
